# EUROPÄISCHE PATENTANMELDUNG

(11) **EP 4 390 465 A1**
(43) Veröffentlichungstag der Anmeldung: **26.06.2024**
(21) Anmeldenummer: 22216412.1
(22) Anmeldetag: 23.12.2022
(51) Int. Cl.: G01T 1/29, A61N 5/10

(54) **VERFAHREN ZUR BERECHNUNG EINER STRAHLENDOSIS**

(71) Anmelder: PTW - Freiburg Physikalisch-Technische Werkstätten Dr. Pychlau GmbH, 79115 Freiburg (DE)
(72) Erfinder: Weidner, Jan, 79108 Freiburg (DE)
(74) Vertreter: Mertzlufft-Paufler, Cornelius

(57) **Zusammenfassung**

Es wird somit ein Verfahren zur Berechnung einer Strahlungsquelle (1) vorgeschlagen, wobei ein zweidimensionales Messergebnis (10) der Strahlungsquelle (1) erzeugt wird, wobei in einem Optimierungsverfahren eine zweidimensionale Größenverteilung (11) einer physikalischen Größe, die strahlungsphysikalisch zu dem Messergebnis (10) korrespondiert, in einer Referenzfläche (12) ermittelt wird. Ferner wird ein Verfahren zur Berechnung einer mit einer Bestrahlung aus einer Strahlungsquelle (1) in einen Körper (4) eingebrachten Strahlendosis vorgeschlagen, wobei in einem erfindungsgemäßen Verfahren zur Berechnung einer Strahlungsquelle (1) eine zweidimensionale Größenverteilung (11) in einer Referenzfläche (12) berechnet wird und wobei aus der zweidimensionalen Größenverteilung (11) mit einem strahlungsphysikalischen Simulationsverfahren eine Dosisverteilung in dem Körper (4) berechnet wird (vgl. Fig. 1) .

## Beschreibung

Die Erfindung betrifft ein Verfahren zur Berechnung einer Strahlungsquelle, wobei ein zweidimensionales Messergebnis der Strahlungsquelle erzeugt wird.

Die Erfindung betrifft weiter ein Verfahren zur Berechnung einer mit einer Bestrahlung aus einer Strahlungsquelle in einen Körper eingebrachten Strahlendosis.

Derartige Verfahren sind besonders relevant bei der Überprüfung und Berechnung von therapeutischen Strahlenbehandlungen für Patienten. Hierbei kann eine Strahlungsquelle an einer Gantry befestigt sein, die um den Patientenkörper bewegt werden kann, um entsprechend der medizinischen Vorgaben eine bestimmte Strahlendosis in den Patienten einzubringen.

Hierbei ist es üblich, vor der Bestrahlung mithilfe eines Detektors zu prüfen, ob die vorgegebene Strahlendosis, insbesondere auch in ihrer räumlichen Verteilung, den gewünschten Vorgaben entspricht. Die Strahlendosis wird insbesondere durch Einstellungen an der Strahlungsquelle, beispielsweise die Stellung von Lamellen zur Begrenzung des Durchlassfeldes und die eingestellte Intensität der Strahlungsquelle bestimmt.

Naturgemäß kann die vom Patienten erhaltene Strahlendosis und deren räumliche Verteilung von der mittels eines Detektors im Vorfeld detektierten Strahlendosis abweichen.

Aufgabe der Erfindung ist es daher, Verfahren zu Berechnungen einer Strahlungsquelle bzw. einer Strahlendosis bereitzustellen, mit Hilfe derer die vom Patienten erhaltene Strahlendosis genauer bestimmt werden kann.

Zur Lösung der genannten Aufgabe sind erfindungsgemäß die Merkmale des Anspruchs 1 vorgesehen. Insbesondere wird somit zur Lösung der genannten Aufgabe bei Verfahren der eingangs beschriebenen Art vorgeschlagen, dass in einem Optimierungsverfahren eine zweidimensionale Größenverteilung einer physikalischen Größe, die strahlungsphysikalisch zu dem Messergebnis korrespondiert, in einer Referenzfläche ermittelt wird.

Auf diese Weise kann aus dem Messergebnis eine Beschreibung des diesem zugrundeliegenden strahlenphysikalischen Prozesses konstruiert werden. Diese Beschreibung besteht hierbei in einer zweidimensionalen Größenverteilung einer physikalischen Größe.

Bei der Größenverteilung kann es sich beispielsweise um eine hypothetische Stellung von Lamellen an der Strahlungsquelle handeln. Es kann sich bei der Größenverteilung auch um eine Verteilung der Intensitäten des einfallenden Strahles handeln.

Die Größenverteilung kann für strahlenphysikalische Berechnungen herangezogen werden, für die das Messergebnis allein nicht herangezogen werden könnte und/oder für die die Verwendung des Messergebnisses einen unverhältnismäßigen Aufwand bedeuten würde. Auch das Heranziehen von realen und mit Unsicherheiten behafteten Einstellungen der Strahlenquelle zur Berechnung des Strahlenganges sind somit verzichtbar. Die Berechnung des Strahlenganges von der Strahlenquelle aus wird beispielsweise mittels Monte-Carlo-Simulationen ausgeführt, welche einen großen Rechen- und somit Zeitaufwand bedeuten, welcher im Rahmen des erfindungsgemäßen Verfahrens verzichtbar ist.

Bei einer vorteilhaften Ausgestaltung des Verfahrens kann vorgesehen sein, dass die Referenzfläche in einem Strahlengang zwischen einem Strahlungsaustritt der Strahlungsquelle und einem das zweidimensionale Messergebnis erzeugenden Detektor oder in einer rückwärtigen oder vorwärtigen Verlängerung des Strahlengangs angeordnet wird.

Auf diese Weise ist die Referenzfläche vorteilhaft positionierbar, um bestehende, beispielsweise lineare Zusammenhänge zwischen der Größenverteilung in der Referenzfläche und der tatsächlichen Größenverteilung an dem Detektor zu Berechnungen heranzuziehen.

Bei einer vorteilhaften Ausgestaltung kann vorgesehen sein, dass die Referenzfläche so ausgebildet und angeordnet wird, dass sie von jedem Strahl zwischen der Strahlungsquelle und dem oder einem das zweidimensionale Messergebnis erzeugenden Detektor höchstens einmal, insbesondere genau einmal, getroffen wird.

Auf diese Weise ist die Berücksichtigung von mehrfach auftreffenden Strahlen verzichtbar. Das Berechnungsverfahren wird somit vereinfacht.

Bei einer vorteilhaften Ausgestaltung kann vorgesehen sein, dass die Referenzfläche eben ist. Alternativ oder zusätzlich kann vorgesehen sein, dass die Referenzfläche senkrecht zu einer Mittelachse der Strahlengangs ausgerichtet ist.

Es hat sich als das Rechenverfahren vereinfachend erwiesen, die Referenzfläche eben und senkrecht zu einer Mittelachse des Strahlengangs auszurichten.

Bei einer vorteilhaften Ausgestaltung kann vorgesehen sein, dass in dem Optimierungsverfahren eine zweidimensionale Größenverteilung der physikalischen Größe auf einen Ort, an dem das zweidimensionale Messergebnis erzeugt wurde, propagiert wird. Zusätzlich kann vorgesehen sein, dass die propagierte Größenverteilung mit dem Messergebnis verglichen wird, um eine verbesserte Größenverteilung zu erzeugen. Zusätzlich kann vorgesehen sein, dass die verbesserte Größenverteilung für eine iterative Wiederholung im Optimierungsverfahren erzeugt wird.

Auf diese Weise kann das Ergebnis des Optimierungsverfahrens qualitativ aufgewertet werden. So kann beispielsweise die Referenzfläche vorteilhaft und nicht in der Detektorebene positioniert sein, wobei dennoch ein Vergleich zwischen der Größenverteilung an der Referenzfläche und dem Messergebnis in der Detektorebene möglich ist und zur Verbesserung der Größenverteilung herangezogen werden kann.

Bei einer vorteilhaften Ausgestaltung kann vorgesehen sein, dass die physikalische Größe so gewählt wird, dass sie über eine lineare Abbildung mit dem Messergebnis zusammenhängt.

Das Optimierungsverfahren wird besonders vereinfacht, wenn eine lineare Abbildung verwendet werden kann.

Bei einer vorteilhaften Ausgestaltung kann vorgesehen sein, dass als die physikalische Größe eine mit einer Fluenz korrelierende Grüße verwendet wird. Hierbei kann es sich um die Fluenz handeln.

Auf diese Weise kann die Berechnung in Zusammenhang mit dem tatsächlichen, physikalischen Verfahren gebracht werden.

Bei einer vorteilhaften Ausgestaltung kann vorgesehen sein, dass die physikalische Größe eindimensional ist.

Auch dies vereinfacht die Berechnungen im Rahmen des Verfahrens.

Bei einer vorteilhaften Ausgestaltung kann vorgesehen sein, dass ein Parameterraum des Optimierungsverfahrens durch mögliche Werte der physikalischen Größe an Orten der Referenzfläche aufgespannt wird. Zusätzlich kann vorgesehen sein, dass hierbei die Werte der physikalischen Größe für jeden Ort der Referenzfläche variiert werden.

Somit kann ein Parameterraum des Optimierungsverfahrens direkt in Zusammenhang gebracht werden mit realistischen, tatsächlich auftretenden Werten der physikalischen Größe. Somit kann der Parameterraum auf technisch sinnvolle Werte eingeschränkt werden, was die Berechnungen weiter vereinfacht.

Bei einer vorteilhaften Ausgestaltung kann vorgesehen sein, dass die berechnete Größenverteilung zur Charakterisierung der Strahlungsquelle verwendet wird. Zusätzlich kann vorgesehen sein, dass es sich bei der Charakterisierung der Strahlungsquelle um eine Kontrolle eine Strahlungsintensität und/oder einer Stellposition von Lamellen an dem oder einem Strahlungsaustritt der Strahlungsquelle handelt.

Auf diese Weise ist es möglich, das Ergebnis des Verfahrens für eine Charakterisierung der Strahlungsquelle und insbesondere eine der genannten Überprüfungen zu verwenden.

Bei einer vorteilhaften Ausgestaltung kann vorgesehen sein, dass das Messergebnis für unterschiedliche Ausrichtungen der Strahlungsquelle zu einem Körper aufgenommen und zu jedem Messergebnis eine Größenverteilung berechnet wird, wobei die Referenzfläche fest zu der Strahlungsquelle ausgerichtet bleibt.

Auf diese Weise können Messergebnisse für unterschiedliche Ausrichtungen der Strahlungsquelle aufgenommen werden, wobei die geometrischen Verhältnisse in Bezug auf die Ausrichtung der Referenzfläche und die daran anschließende Berechnung gleich bleiben. Auch dies vereinfacht das erfindungsgemäße Verfahren.

Bei einer vorteilhaften Ausgestaltung kann vorgesehen sein, dass der oder ein das Messergebnis erzeugende Detektor und die Strahlungsquelle an einer Gantry angeordnet und gemeinsam zur Aufnahme mehrerer Messergebnisse verfahren werden.

Auf diese Weise kann auf einfache Art und Weise eine große Anzahl von Messergebnissen zur Durchführung des erfindungsgemäßen Verfahrens aufgenommen werden.

Bei einer vorteilhaften Ausgestaltung kann vorgesehen sein, dass die Strahlungsquelle nach einem Bestrahlungsplan angesteuert und verfahren wird.

Das erfindungsgemäße Verfahren ist somit zur Therapie von Patienten, wobei Bestrahlungspläne verwendet werden, geeignet.

Bei einer vorteilhaften Ausgestaltung kann vorgesehen sein, dass zwischen zwei Optimierungsschritten des Optimierungsverfahrens die jeweilige Größenverteilung geglättet wird.

Somit können beispielsweise Informationen darüber, inwieweit ein Strahlprofil in sich Unregelmäßigkeiten aufweisen kann, zur Nachberechnung einer jeweiligen Größenverteilung genutzt werden.

Bei einer vorteilhaften Ausgestaltung kann vorgesehen sein, dass jede Größenverteilung zumindest auf einem Teil der Referenzfläche berechnet wird, der bei einer vollständigen Öffnung des oder eines Strahlungsaustritts der Strahlungsquelle bestrahlt würde.

Somit wird die Größenverteilung im größtmöglichen, relevanten Bereich der Referenzfläche berechnet, sodass bei jeder möglichen Öffnungseinstellung der Strahlungsquelle eine flächenmäßig vollständige Erfassung der Strahlen, insbesondere aller Teilstrahlen, erfolgt.

Alternativ oder zusätzlich sind zur Lösung der genannten Aufgabe erfindungsgemäß die Merkmale des nebengeordneten, auf ein Verfahren zur Berechnung einer mit einer Bestrahlung aus einer Strahlungsquelle in einen Körper eingebrachten Strahlendosis gerichteten Anspruchs vorgesehen. Insbesondere wird somit zur Lösung der genannten Aufgabe bei Verfahren der eingangs beschriebenen Art erfindungsgemäß vorgeschlagen, dass in einem erfindungsgemäßen Verfahren zur Berechnung einer Strahlungsquelle eine zweidimensionale Größenverteilung in einer Referenzfläche berechnet wird, und dass aus der zweidimensionalen Größenverteilung mit einem strahlenphysikalischen Simulationsverfahren eine Dosisverteilung in dem Körper berechnet wird.

Somit können die Vorteile des erfindungsgemäßen Verfahrens zur Berechnung einer Strahlungsquelle bei strahlungsphysikalischen Simulationsverfahren, beispielsweise bereits bekannten strahlungsphysikalischen Simulationsverfahren, verwendet werden und somit auch im Rahmen einer Therapie von Patienten.

Bei einer vorteilhaften Ausgestaltung kann vorgesehen sein, dass das Simulationsverfahren wenigstens eine Monte-Carlo-Simulation ausführt.

Auf diese Weise können in dem Simulationsverfahren zufällige Größen berechnet werden.

Die Erfindung wird nun anhand eines Ausführungsbeispiels näher beschrieben, ist jedoch nicht auf das Ausführungsbeispiel beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigt:

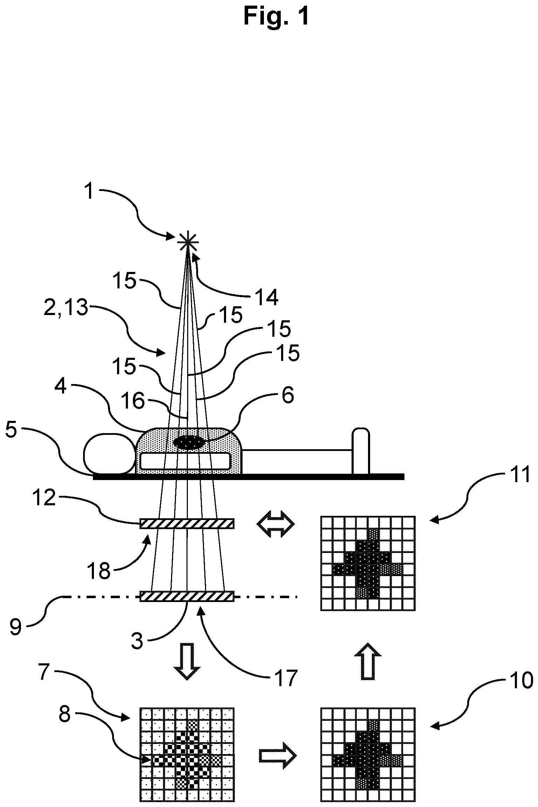

- Figur 1: eine schematische Darstellung der Ausführung der erfindungsgemäßen Verfahren.

Figur 1 zeigt in einer schematischen Darstellung eine Strahlungsquelle 1, von der ein aus einzelnen Strahlen 15 bestehender Gesamtstrahl 2 ausgeht, der auf einen Detektor 3 trifft. Der Gesamtstrahl 2 weist einen Strahlengang 13 auf, welcher eine Mittelachse 16 aufweist.

Ein Körper 4 eines Patienten auf einem Patiententisch 5 ist optional und insbesondere dann anwesend, wenn das erfindungsgemäße Verfahren im Rahmen einer Strahlentherapie genutzt werden soll. In diesem Fall ist innerhalb des Körpers 4 des Patienten ein Therapiebereich 6 festgelegt, in den beispielsweise eine bestimmte Strahlendosis eingebracht werden soll. Diese kann sich beispielsweise aus einem Behandlungsplan ergeben.

Der Detektor 3 gibt ein Rohbild 7 aus, wobei es sich um ein Strahlprofil 8 des Gesamtstrahles 2 in einer Detektorebene 9 handelt. Hieraus lässt sich ein zweidimensionales Messergebnis 10 berechnen. Hierbei kann es sich beispielsweise um ein Bild einer Strahlendosis handeln.

Im Rahmen des erfindungsgemäßen Verfahrens zur Berechnung einer Strahlungsquelle 1, wobei ein zweidimensionales Messergebnis 10 der Strahlungsquelle 1 erzeugt wird, wird in einem Optimierungsverfahren eine zweidimensionale Größenverteilung 11 einer physikalischen Größe, die strahlenphysikalisch zu dem Messergebnis 10 korrespondiert, in einer Referenzfläche 12 vermittelt. Hierbei ist die Referenzfläche 12 in dem Strahlengang 13 zwischen einem Strahlungsaustritt 14 der Strahlungsquelle 1 und einem das zweidimensionale Messergebnis 10 erzeugenden Detektor 3 angeordnet. In einer nicht gezeigten Ausführungsformen kann die Referenzfläche auch in einer rückwärtigen oder vorwärtigen Verlängerung des Strahlengangs 13 angeordnet sein.

Die Referenzfläche 12 ist ferner so ausgebildet und angeordnet, dass sie von jedem Strahl 15 zwischen der Strahlungsquelle 1 und dem das zweidimensionale Messergebnis 10 erzeugenden Detektor 3 höchstens einmal, nämlich genau einmal, getroffen wird.

Die Referenzfläche 12 ist im Ausführungsbeispiel eben und senkrecht zu einer Mittelachse 16 des Strahlengangs 13 ausgerichtet.

In dem Optimierungsverfahren wird eine zweidimensionale Größenverteilung 11 der physikalischen Größe auf einen Ort 17, an dem das zweidimensionale Messergebnis 10 erzeugt wurde, propagiert. Der Ort 17 ist hierbei die Position des Detektors 3 in der Detektorebene 9. Hierbei wird die propagierte Größenverteilung mit dem Messergebnis 10 verglichen, um für eine iterative Wiederholung im Optimierungsverfahren eine verbesserte Größenverteilung zu erzeugen.

In dem dargestellten Verfahren ist außerdem die physikalische Größe so gewählt, dass sie über eine lineare Abbildung mit dem Messergebnis 11 zusammenhängt. Als physikalische Größe wird hierbei eine mit einer Fluenz korrelierende Größe, nämlich die Fluenz selbst, verwendet. Die verwendete physikalische Größe ist eindimensional.

Ferner wird im Rahmen des dargestellten Verfahrens ein Parameterraum des Optimierungsverfahrens durch mögliche Werte der physikalischen Größe an Orten 18 der Referenzfläche 12 aufgespannt, wobei die Werte der physikalischen Größe für jeden Ort 18 der Referenzfläche variiert werden.

Die berechnete Größenverteilung wird ferner zu einer Charakterisierung der Strahlungsquelle 1 verwendet, nämlich zu einer Kontrolle einer Strahlungsintensität und zu einer Kontrolle einer Stellposition von Lamellen an dem Strahlungsaustritt 14 der Strahlungsquelle 1.

Das dargestellte Verfahren kann auch für unterschiedliche Ausrichtungen der Strahlungsquelle 1 zu dem Körper 4 ausgeführt werden, wobei jeweils Messergebnisse 10 aufgenommen werden und wobei zu jedem Messergebnis 10 eine Größenverteilung 11 berechnet wird, wobei die Referenzfläche 12 fest zu der Strahlungsquelle 1 ausgerichtet bleibt.

Hierbei können in einer nicht gezeigten Ausführungsform der das Messergebnis 10 erzeugende Detektor 3 und die Strahlungsquelle 1 an einer Gantry angeordnet sein und gemeinsam zur Aufnahme der Messergebnisse 10 verfahren werden. Die Strahlungsquelle 1 kann hierbei nach einem Bestrahlungsplan gesteuert und verfahren werden.

Ferner wird zwischen zwei Optimierungsschritten des Optimierungsverfahren die jeweilige Größenverteilung 11 geglättet. Im Rahmen des schematisch dargestellten Verfahrens wird außerdem jede Größenverteilung 11 zumindest auf einem Teil der Referenzfläche 12 berechnet, der bei einer vollständigen Öffnung des Strahlungsaustritt 14 der Strahlungsquelle 1 bestrahlt würde.

Die schematische Darstellung zeigt weiter ein erfindungsgemäßes Verfahren zur Berechnung einer mit einer Bestrahlung aus einer Strahlungsquelle 1 in einen Körper 4 eingebrachten Strahlendosis, wobei in einem erfindungsgemäßen Verfahren eine zweidimensionale Größenverteilung 11 in einer Referenzfläche 12 berechnet wird und wobei aus der zweidimensionalen Größenverteilung 11 mit einem strahlungsphysikalischen Simulationsverfahren eine Dosisverteilung in dem Körper 4 berechnet wird. Dies ist die Dosisverteilung in dem Therapiebereich 6. Das Simulationsverfahren führt außerdem wenigstens eine Monte-Carlo-Simulation aus.

Es wird somit ein Verfahren zur Berechnung einer Strahlungsquelle 1 vorgeschlagen, wobei ein zweidimensionales Messergebnis 10 der Strahlungsquelle 1 erzeugt wird, wobei in einem Optimierungsverfahren eine zweidimensionale Größenverteilung 11 einer physikalischen Größe, die strahlungsphysikalisch zu dem Messergebnis 10 korrespondiert, in einer Referenzfläche 12 ermittelt wird. Ferner wird ein Verfahren zur Berechnung einer mit einer Bestrahlung aus einer Strahlungsquelle 1 in einen Körper 4 eingebrachten Strahlendosis vorgeschlagen, wobei in einem erfindungsgemäßen Verfahren zur Berechnung einer Strahlungsquelle 1 eine zweidimensionale Größenverteilung 11 in einer Referenzfläche 12 berechnet wird und wobei aus der zweidimensionalen Größenverteilung 11 mit einem strahlungsphysikalischen Simulationsverfahren eine Dosisverteilung in dem Körper 4 berechnet wird.

### Bezugszeichenliste

- 1: Strahlungsquelle
- 2: Gesamtstrahl
- 3: Detektor
- 4: Körper
- 5: Patiententisch
- 6: Therapiebereich
- 7: Rohbild
- 8: Strahlprofil
- 9: Detektorebene
- 10: Messergebnis
- 11: Größenverteilung
- 12: Referenzfläche
- 13: Strahlengang
- 14: Strahlungsaustritt
- 15: Strahl
- 16: Mittelachse
- 17: Ort
- 18: Ort

## Patentansprüche

1. Verfahren zur Berechnung einer Strahlungsquelle (1), wobei ein zweidimensionales Messergebnis (10) der Strahlungsquelle (1) erzeugt wird, **dadurch gekennzeichnet, dass** in einem Optimierungsverfahren eine zweidimensionale Größenverteilung (11) einer physikalischen Größe, die strahlungsphysikalisch zu dem Messergebnis (10) korrespondiert, in einer Referenzfläche (12) ermittelt wird.

2. Verfahren nach Anspruch 1, **dadurch gekennzeichnet, dass** die Referenzfläche (12) in einem Strahlengang (13) zwischen einem Strahlungsaustritt (14) der Strahlungsquelle (1) und einem das zweidimensionale Messergebnis (10) erzeugenden Detektor (3) oder in einer rückwärtigen oder vorwärtigen Verlängerung des Strahlengangs (13) angeordnet wird.

3. Verfahren nach einem der vorangehenden Ansprüche, **dadurch gekennzeichnet, dass** die Referenzfläche (12) so ausgebildet und angeordnet wird, dass sie von jedem Strahl (15) zwischen der Strahlungsquelle (1) und dem oder einem das zweidimensionale Messergebnis (10) erzeugenden Detektor (3) höchstens einmal, insbesondere genau einmal, getroffen wird.

4. Verfahren nach einem der vorangehenden Ansprüche, **dadurch gekennzeichnet, dass** die Referenzfläche (12) eben ist und/oder dass die Referenzfläche (12) senkrecht zu einer Mittelachse (16) des Strahlengangs (13) ausgerichtet ist.

5. Verfahren nach einem der vorangehenden Ansprüche, **dadurch gekennzeichnet, dass** in dem Optimierungsverfahren eine zweidimensionale Größenverteilung (11) der physikalischen Größe auf einen Ort (17), an dem das zweidimensionale Messergebnis (10) erzeugt wurde, propagiert wird, insbesondere wobei die propagierte Größenverteilung (11) mit dem Messergebnis (10) verglichen wird, um, vorzugsweise für eine iterative Wiederholung im Optimierungsverfahren, eine verbesserte Größenverteilung (11) zu erzeugen.

6. Verfahren nach einem der vorangehenden Ansprüche, **dadurch gekennzeichnet, dass** die physikalische Größe so gewählt wird, dass sie über eine lineare Abbildung mit dem Messergebnis (10) zusammenhängt.

7. Verfahren nach einem der vorangehenden Ansprüche, **dadurch gekennzeichnet, dass** als die physikalische Größe eine mit einer Fluenz korrelierende Größe, insbesondere die Fluenz, verwendet wird.

8. Verfahren nach einem der vorangehenden Ansprüche, **dadurch gekennzeichnet, dass** die physikalische Größe eindimensional ist.

9. Verfahren nach einem der vorangehenden Ansprüche, **dadurch gekennzeichnet, dass** ein Parameterraum des Optimierungsverfahrens durch mögliche Werte der physikalischen Größe an Orten (18) der Referenzfläche (12) aufgespannt wird, insbesondere wobei die Werte der physikalischen Größe für jeden Ort (18) der Referenzfläche (12) variiert werden.

10. Verfahren nach einem der vorangehenden Ansprüche, **dadurch gekennzeichnet, dass** die berechnete Größenverteilung (11) zu einer Charakterisierung der Strahlungsquelle (1), insbesondere zu einer Kontrolle einer Strahlungsintensität und/oder einer Stellposition von Lamellen an dem oder einem Strahlungsaustritt (14) der Strahlungsquelle (1), verwendet wird.

11. Verfahren nach einem der vorangehenden Ansprüche, **dadurch gekennzeichnet, dass** das Messergebnis (10) für unterschiedliche Ausrichtungen der Strahlungsquelle (1) zu einem Körper (4) aufgenommen und zu jedem Messergebnis (10) eine Größenverteilung (11) berechnet wird, wobei die Referenzfläche (12) fest zu der Strahlungsquelle (1) ausgerichtet bleibt.

12. Verfahren nach einem der vorangehenden Ansprüche, **dadurch gekennzeichnet, dass** der oder ein das Messergebnis (10) erzeugender Detektor (3) und die Strahlungsquelle (1) an einer Gantry angeordnet und gemeinsam zur Aufnahme mehrerer Messergebnisse (10) verfahren werden.

13. Verfahren nach einem der vorangehenden Ansprüche, **dadurch gekennzeichnet, dass** die Strahlungsquelle (1) nach einem Bestrahlungsplan angesteuert und verfahren wird.

14. Verfahren nach einem der vorangehenden Ansprüche, **dadurch gekennzeichnet, dass** zwischen zwei Optimierungsschritten des Optimierungsverfahrens die jeweilige Größenverteilung (11) geglättet wird.

15. Verfahren nach einem der vorangehenden Ansprüche, **dadurch gekennzeichnet, dass** jede Größenverteilung (11) zumindest auf einem Teil der Referenzfläche (12) berechnet wird, der bei einer vollständigen Öffnung des oder eines Strahlungsaustritts (14) der Strahlungsquelle (1) bestrahlt würde.

16. Verfahren zur Berechnung einer mit einer Bestrahlung aus einer Strahlungsquelle (1) in einen Körper (4) eingebrachten Strahlendosis, wobei in einem Verfahren nach einem der vorangehenden Ansprüche eine zweidimensionale Größenverteilung (11) in einer Referenzfläche (12) berechnet wird und wobei aus der zweidimensionalen Größenverteilung (11) mit einem strahlungsphysikalischen Simulationsverfahren eine Dosisverteilung in dem Körper (4) berechnet wird.

17. Verfahren nach dem vorangehenden Anspruch, **dadurch gekennzeichnet, dass** das Simulationsverfahren wenigstens eine Monte-Carlo-Simulation ausführt.
